(19) Europäisches Patentamt
European Patent Office
Office européen des brevets

(11) EP 1 951 377 B1

(12) **EUROPEAN PATENT SPECIFICATION**

(45) Date of publication and mention of the grant of the patent:
**03.06.2009 Bulletin 2009/23**

(21) Application number: **06821185.3**

(22) Date of filing: **11.10.2006**

(51) Int Cl.:
***A61Q 19/08*** *(2006.01)* ***A61K 8/72*** *(2006.01)*

(86) International application number:
**PCT/IB2006/053738**

(87) International publication number:
**WO 2007/046038 (26.04.2007 Gazette 2007/17)**

(54) **COSMETIC METHOD FOR USE IN SMOOTHING THE SKIN**

KOSMETISCHE VERFAHREN ZUR VERWENDUNG BEI DER HAUTGLÄTTUNG

PROCEDE COSMETIQUE DESTINE A LISSER LA PEAU

(84) Designated Contracting States:
**AT BE BG CH CY CZ DE DK EE ES FI FR GB GR HU IE IS IT LI LT LU LV MC NL PL PT RO SE SI SK TR**

(30) Priority: **21.10.2005 FR 0553213**
**29.11.2005 US 740286 P**

(43) Date of publication of application:
**06.08.2008 Bulletin 2008/32**

(73) Proprietor: **L'Oréal**
**75008 Paris (FR)**

(72) Inventor: **CASSIN, Guillaume**
**F-91140 Villebon Sur Yvette (FR)**

(74) Representative: **Tanty, François**
**Nony & Associés**
**3, rue de Penthièvre**
**75008 Paris (FR)**

(56) References cited:
**EP-A- 1 038 519**
**EP-A- 1 090 627**
**EP-A- 1 262 168**
**EP-A- 1 566 171**
**WO-A-20/05046626**
**FR-A- 2 843 025**
**FR-A- 2 867 681**
**US-A1- 2004 142 008**

EP 1 951 377 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

The present invention relates to a cosmetic skin care method, comprising the topical application to the skin of a composition combining a synthetic polymer tightening agent and fibers.
In the course of the aging process, a change in the structure and functions of the skin appears. The main clinical signs observed are the appearance of wrinkles and fine lines linked to a slackening of the skin. Those skilled in the art know that such a slackening may be corrected immediately by the application of tightening agent to the skin.
To date, the use of numerous tightening agents for treating wrinkles is known to those skilled in the art. In particular, certain synthetic polymers are considered (EP 1 038 519, WO 98/29092, for 2 843 025, WO 03/086342, EP 1 566 171, FR 2 863 494). Unfortunately, compositions containing such tightening agents, despite having a highly satisfactory tightening effect, have the mayor drawback of having an effect that is limited over time.
As a consequence, there is a great need for cosmetic compositions having both excellent effectiveness and a long-lasting tightening effect:
The applicant has discovered that the inclusion of fibers into compositions comprising a synthetic polymer tightening agent improves the mechanical properties of the polymer tightening film by allowing it especially to follow facial expressions without cracking, and thus improving its persistence.
The subject of the present invention is therefore a cosmetic skin care method for smoothing human skin on the face and/or body and/or to diminish or remove the signs of skin aging, in particular to reduce or remove wrinkles and/or fine lines from the skin comprising, the topical application to the skin of a composition comprising, in a physiologically acceptable medium, at least:

- one synthetic polymer tightening agent, and
- fibers.

The use of fibers in cosmetics is disclosed in numerous documents. Mention may be made, among others, of the documents EP 1 090 626, EP 1 090 627, EP 1 092 424, EP 1 243 251 and EP 1 262 168. However, in none of these documents are synthetic polymers with a tightening effect associated with fibers, in order to correct the signs of aging.
The method according to the invention is a method for smoothing human skin on the face and/or body and/or to diminish or remove the signs of skin aging, in particular to reduce or remove wrinkles and/or fine lines from the skin.
The tightening and persistence effects are especially able to be measured by scoring photographs taken at various times after application of a product to an area having wrinkles. The technique called fringe projection that finely quantifies the microrelief changes induced by a product can also be used.
The term "physiologically acceptable medium" is understood to mean a medium devoid of toxicity and compatible with skin and possibly with its integuments, mucous membranes and semimucous membranes.
The term "glass transition temperature", having the abbreviation Tg, is understood to mean the temperature below which the polymer becomes rigid. When the temperature increases, the polymer passes through a transition state that allows the macromolecular chains to slide relative to each other and the polymer softens.
In the context of the present invention, the protocol for measuring the glass transition temperature of copolymers or homopolymers formed from monomers used for the preparation of the copolymers uses DSC (Differential Scanning Colorimetry) characterization and is given in detail hereinbelow:
Film transitions (glass transitions, melting transitions) are studied by DSC based on 2 heating/cooling cycles at 10°C/min between -140°C and 130°C (around 2 hours). The measurements are carried out under nitrogen flushing and using hermetically-sealed pans in order not to change the film composition by evaporation of the solvent during the DSC test:

- apparatus: DSC 2920 from TA Instruments
- purge gas: Alphagaz nitrogen 2 to 50 ml/min
- crucible: 50 μl crimped stainless steel pan from Perkin Elmer
- energy and temperature calibration: melting point of indium
- test sample: conditioned by drying (around 10 mg)
- heat treatments:

  1. CO: cooling from +25°C to -140°C at 10°C/min
  2. CO (a): conditioning at -140°C
  3. H1: heating from -140°C to +130°C at 10°C/min
  4. C1: cooling from +130°C to -140°C at 10°C/mm
  5. C1 (a): conditioning at -140°C
  6. H2: heating from -140°C to + 130°C at -10°C/min.

Two samples are studied for each product.

In the context of the present invention, the expressions "comprised between ... and ...", "varying between ... and ..." or "ranging from ... to ..." mean that the limits are also included.

As stated previously, the compositions according to the invention comprise at least one synthetic polymer tightening agent.

## SYNTHETIC POLYMER TIGHTENING AGENT

The term "tightening agent" is understood to mean a compound capable of having a tightening effect, that is to say being able to tighten the skin and by this tightening effect to smooth the skin and make wrinkles and fine lines diminish or even disappear immediately.

The term "synthetic polymer" characterizes any polymer obtained chemically or by production in a system with the constituents necessary for this production.

This synthetic polymer tightening agent may be in the form of a mixture of such synthetic polymer tightening agent.

More particularly, the term "synthetic polymer tightening agent" may be understood to mean any synthetic polymer producing, at a concentration of 7 wt% in water or any physiologically acceptable medium, a shrinkage of the isolated *stratum corneum* of at least 0.9%, or even of more than 1%, at 30°C and under 40% relative humidity, depending on the following method for measuring the tightening effect.

The principle behind this method consists in measuring the length of a test specimen of *stratum corneum* isolated from human skin originating from a surgical operation, before and after treatment with the prospective tightening agent.

To do this, the test specimen is placed between the two jaws of the device, one of which is stationary and the other is movable, in an atmosphere at 30°C and 40% relative humidity. A pull force is exerted on the test specimen, and the curve of the force (in grams) is recorded as a function of the length (in millimeters), the zero length corresponding to the contact between the two jaws of the device. The tangent to the curve in its linear region is subsequently plotted. The intersection of this tangent with the x-axis corresponds to the apparent length $L_0$ of the test specimen at zero force. The test specimen is subsequently relaxed and then 2 mg/cm$^2$ of the test composition (7 wt% solution of the tightening agent under consideration) are applied to the *Stratum corneum.* After drying for 15 5 minutes, the above steps are again carried out in order to determine the length $L_1$ of the test specimen after treatment. The percentage shrinkage is defined by: % shrinkage = $100 \times (L_1-L_0)/L_0$. To characterize a tightening effect, this percentage must be negative and the tightening effect is greater the higher the absolute value of the percentage shrinkage.

Aside from the fact that they are, in general, dispersed in water for carrying out the aforesaid test, the synthetic polymer tightening agents may be, in the composition used according to the invention, either in solution or in suspension in a polar or apolar liquid, or in dry form able to be redispersed in a cosmetic solvent.

The synthetic polymer tightening agents used according to the invention, may comprise at least polyurethane polymers and copolymers, in particular polyester/polyurethane copolymers or polyether/polyurethane copolymers; acrylic polymers and copolymers; or grafted silicone polymers.

These tightening agents may especially be in the form of interpenetrating polymer networks (IPNs), polycondensates, block polymers or star polymers.

### Interpenetrating polymer network.

According to a first variant, the composition according to the present invention comprises at least one interpenetrating polymer network (IPN) type synthetic polymer tightening agent.

The term "interpenetrating polymer network" in the sense of the present invention is understood to mean a blend of two entangled polymers, obtained by simultaneous polymerization and/or crosslinking of two types of monomer, the blend obtained having a single glass transition temperature.

Examples of IPNs that are suitable for use in the present invention, and also the process for preparing them, are for example described in patents US 6 139 322 and US 6 465 001.

Preferably, the IPN according to the invention comprises at least one polyacrylic polymer and, where appropriate, at least one polyurethane or one vinylidene fluoride/hexafluoropropylene copolymer.

According to a preferred embodiment, the IPN according to the invention comprises a polyurethane polymer and a polyacrylic polymer. Such IPNs are especially those of the HYBRIDUR® series that are commercially available from Air Products.

An IPN that is particularly preferred is in the form of an aqueous dispersion of particles having a weight-average size of between 90 and 110 nm and a number-average size of about 80 nm. This IPN preferably has a glass transition temperature, Tg, ranging from about -60°C to +100°C. An IPN of this type is especially sold by Air Products under the

trademark HYBRIDUR X-01602®. Another IPN that is suitable for use in the present invention is referenced HYBRIDUR X18693-21®.

Other IPNs that are suitable for use in the present invention comprise IPNs consisting' of a blend of a polyurethane with a vinylidene fluoride/hexafluoropropylene copolymer. These IPNs may especially be prepared as described, in patent US 5 349 003. As a variant, they are commercially available in the form of a colloidal dispersion in water, in a ratio of the fluorocopolymer to the acrylic polymer of between 70:30 and 75:25, under the trademarks KYNAR RC-10147® and KYNAR RC-10151® from Atofina.

Polycondensate

The composition may according to a second variant comprise by way of synthetic polymer tightening agents at least one polymer in the form of a polycondensate.

Polymers, in the form of polycondensates having an antiwrinkle effect, have especially been described in patent WO 98/29092.

As polycondensates, mention may be made of anionic, cationic, nonionic or amphoteric polyurethanes, polyurethane/acrylics, polyurethane/poyvinylpyrrodones, polyester/polyurethanes, polyether/polyurethanes, polyureas, and blends thereof.

The polyurethane may be, for example, an aliphatic, cycloaliphatic or aromatic polyurethane, polyurea/utethane or polyurea copolymer, comprising singly or as a blend:

- at least one block of linear or branched aliphatic and/or cycloaliphatic and/or aromatic polyester origin; and/or
- at least one block of aliphatic and/or cycloaliphatic and/or aromatic polyether origin; and/or
- at least one block comprising fluoro groups.

The polyurethanes may also be obtained from polyesters, whether branched or not, or from alkyds comprising mobile hydrogen atoms that are modified by the reaction between a diisocyanate and a bifunctional organic compound (for, example dihydro, diamino or hydroxyamino), comprising in addition either a carboxylic acid or carboxylate group, or a sulfonic acid or sulfonate group, or even a tertiary amine group that can be neutralized or a quaternary ammonium group. Mention may also be made of polyesters, polyester amides, fatty chain polyesters, polyamides, and epoxy esters resins.

With a view to forming a polyurethane, dimethylolpropionic acid, trimellitic acid or a derivative such as trimellitic anhydride, the sodium salt of 3-sulfopentanediol acid and the sodium salt of 5-sulfo-1,3-benzenedicarboxylic acid may be mentioned as the anionic carrier monomer that may be used during the polycondensation.

Acrylic polymers and copolymers may also be mentioned.

Among the polycondensates, mention may be made of the polymers sold under the trademarks AVALURE UR405® and AVALURE UR450® by Noveon.

Grafted silicone polymer

Among the synthetic polymer tightening agents used in the composition according to the invention, mention may especially, as a variant, be made of grafted silicone polymers as defined in patent EP1 038 519. It may be, more particularly, a polymer comprising a main silicone or polysiloxane (Si-O- polymer) chain onto which is grafted, within said chain and also possibly at one at least of its ends, at least one nonsilicone organic group.

The polymers having a polysiloxane backbone grafted by nonsilicone organic monomers according to the invention may be existing commercial products, or else they may be obtained according to any means known to those skilled in the art, in particular by reaction between (i) a starting silicone correctly functionalized on one or more of its silicon atoms and (ii) a nonsilicone organic compound itself correctly functionalized by a functional group that is capable of reacting with the functional group(s) carried by said silicone to form a covalent bond; a conventional example of such a reaction is the hydrosilylation reaction between ≡Si-H groups and $CH_2$=CH- vinyl groups, or else the reaction between -SH thio-functional groups and these same vinyl groups.

Examples of polymers having a polysiloxane backbone grafted by nonsilicone organic monomers suitable for use in the present invention, and also their particular preparation method, are especially described in patent applications EP-A 0 582 152, WO 93/23009 and WO 95/03776.

According to a particularly preferred embodiment of the present invention, the silicone polymer having a polysiloxane backbone grafted by nonsilicone organic monomers results from the radical copolymerization between, on one hand, at least one ethylenically unsaturated nonsilicone anionic organic, monomer and/or an ethylenically unsaturated nonsilicone hydrophobic organic monomer and, on the other hand, a silicone having in its chain at least one functional group capable of reacting with said ethylenic unsaturated groups of said nonsilicone monomers to form a covalent bond,

in particular thio-functional groups.

According to the present invention, said ethylenically unsaturated nonsilicone anionic monomers are preferably chosen, singly or as blends, from linear or branched, unsaturated carboxylic acids, possibly partially or completely neutralized in salt form, this or these unsaturated carboxylic acid(s) may be more particularly acrylic acid, methacrylic acid, maleic acid, maleic anhydride, itaconic acid, fumaric acid and crotonic acid. Suitable salts are especially alkali metal, alkaline-earth metal and ammonium salts. It can be noted that, similarly, in the final grafted silicone polymer, the organic group having an anionic character that comprises the result of the radical (homo)polymerization of at least one unsaturated carboxylic acid type anionic monomer may, after reaction, be post-neutralized with a base (sodium hydroxide, ammonium hydroxide) in order to convert it into salt form.

According to the present invention, the ethylenically unsaturated hydrophobic monomers are preferably chosen, singly or as blends, from acrylic acid esters of alkanols and/or methacrylic acid esters of alkanols. The alkanols are preferably $C_1$-$C_{18}$, and more particularly. $C_1$-$C_{12}$, alkanols. The preferred monomers are chosen from the group composed of isooctyl (meth)acrylate, isononyl (meth)acrylate, 2-ethylhexyl (meth)acrylate, lauryl (meth)acrylate, isopentyl (meth) acrylate, n-butyl (meth)acrylate, isobutyl (meth)acrylate, methyl (meth)acrylate, *tert*-butyl (meth)acrylate, tridecyl (meth) acrylate, stearyl (meth)acrylate or mixtures thereof.

A family of silicone polymers having a polysiloxane backbone grafted by nonsilicone organic monomers that is particularly well suited for use in the present invention is composed of grafted silicone polymers comprising in their structure the unit of formula (I) below:

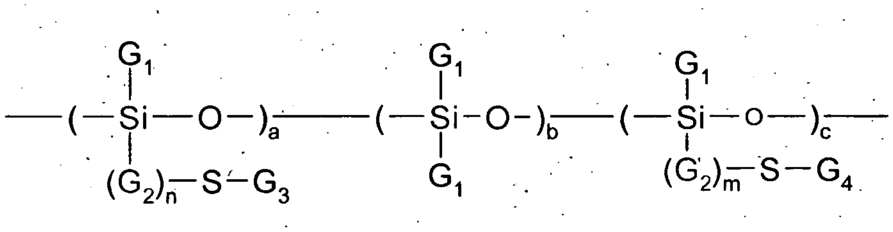

(I)

in which the $G_1$ radicals, which are identical or different, represent hydrogen or a $C_1$-$C_{10}$ alkyl radical or even a phenyl radical; the $G_2$ radicals, which are identical or different, represent a $C_1$-$C_{10}$ alkylene group; $G_3$ represents a polymer residue resulting from the (homo)polymerization of at least one ethylenically unsaturated anionic monomer; $G_4$ represents a polymer residue resulting from the (homo)polymerization of at least one ethylenically unsaturated hydrophobic monomer; m and n are, independently of one another, equal to 0 or 1; a is an integer ranging from 0 to 50; b is an integer that may be between 10 and 350; and c is an integer ranging from 0 to 50, on condition that one of parameters a and c is other than O.

Preferably, the unit of formula (I) above has at least one, and even more preferentially all, of the following characteristics:

- the $G_1$ radicals denote a $C_1$-$C_{10}$-alkyl radical;
- n is hot zero, and the $G_2$ radicals represent a $C_1$-$C_3$ divalent radical;
- $G_3$ represents a polymer radical resulting from the (homo)polymerization of at least one ethylenically unsaturated carboxylic acid, preferably acrylic acid and/or methacrylic acid, type monomer;
- $G_4$ represents a polymer radical resulting from the (homo)polymerization of at least one $C_1$-$C_{10}$ alkyl (meth)acrylate type monomer.

Examples of grafted silicone polymers comprising in their structure the unit of formula (I) are thus especially polydimethylsiloxanes (PDMS) onto which are grafted, via a thiopropylene-type linking chain, mixed polymer units of poly(meth)acrylic acid type and/or of polyalkyl (meth)acrylate type.

These polymers are referenced under the CTFA name "Polysilicone-8".

It may also be a propylthio(polymethyl acrylate)-, propylthio(polymethyl methacrylate)- or propylthio(polymethacrylic acid)-grafted polydimethylsiloxane. As a variant, it may be a propylthio(polyisobutyl methacrylate)- or propylthio (polymethacrylic acid)-grafted polydimethylsiloxane.

Such grafted silicone polymers are especially sold by 3M under the trademarks VS 80®, VS 70® or L021®.

Preferably, the number-average molecular weight of the silicone polymers having a polysiloxane backbone grafted by the nonsilicone organic monomers of the invention varies from 10 000 to about 1 000 000, and still more preferentially from 10 000 to about 100 000.

Star polymer

According to yet another possibility, the synthetic polymer tightening agent that may be used in the composition according to the invention may comprise at least one polymer of "star" structure represented by formula (II) below:

$$A\text{-}[(M_1)p_1 - (M_2)p_2 \ldots (M_i)_{pj}]_n \qquad (II)$$

in which:

- A represents a multifuncfional centre, of functionality "n", n being an integer greater than 2, in particular greater than 5;
- $[(M_1)_{p1} - (M_2)_{p2} \ldots (M_i)_{pj}]$ represents a polymer chain, also called a "branch", consisting of polymerized monomers $M_i$, which are identical or different, having a polymerization index pj, each branch being identical or different, and being covalently grafted onto said centre A; and
- i is greater than or equal to 1, and pj is greater than or equal to 2,

said polymer comprising one or more monomers $M_i$, the corresponding homopolymer of which has a Tg of greater than or equal to 10°C, preferably greater than or equal to 15°C, and better still greater than or equal to 20°C, and this or these monomer(s) $M_i$ bering present in a minimum amount of about 45% by weight, preferably in an amount varying between 55 and 99% by weight, and better still between 75 and 90% by weight relative to the total weight of all the monomers in the final polymer. These polymers, and also the process for preparing them, are for example described in document EP 1 043 345.

Block polymer

As a variant, the synthetic polymer tightening agents that may be used in the composition according to the invention may be block polymers.

The term "block polymer" is understood to mean, in a very general way, a polymer composed of at least two distinct homopolymers or copolymers. The homopolymers may be composed of blocks comprised only of monomers A and B respectively.

A preferred block polymer for use in the present invention is a copolymer comprising units deriving from styrene and units deriving from ethyl (meth)acrylate, in which the weight ratio of the units deriving from styrene to the units deriving from ethyl (meth)acrylate is greater than or equal to 1.

It should be pointed out that the term "unit deriving from styrene" is understood to mean a unit obtained directly from a styrene monomer by polymerization, that is to say, a unit of the formula below:

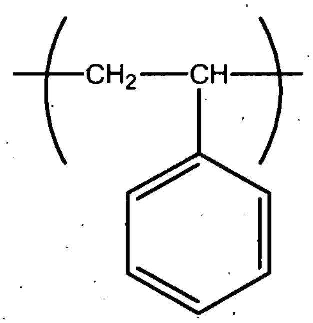

It should be further pointed out that the term "unit deriving from ethyl (meth)acrylate" is understood to mean a unit obtained directly from an ethyl acrylate monomer, in which case the unit is represented by the formula below:

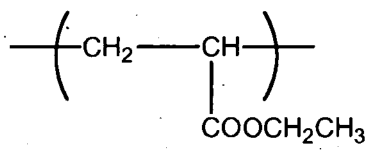

or a unit obtained directly from an ethyl methacrylate monomer, in which case the unit is represented by the formula below:

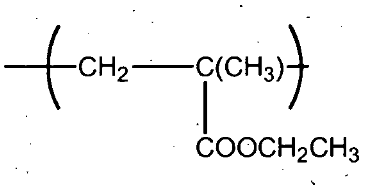

Besides the units deriving from styrene and from ethyl (meth)acrylate, the copolymers as defined hereinabove may comprise units deriving from monomers chosen from (meth)acrylic acid, methyl (meth)acrylate, butyl (meth)acrylate, ethylhexyl (meth)acrylate and 2-hydroxyethyl (meth)acrylate.

Advantagously, the block copolymers used according to the invention are linear block copolymers of the A-[B-A]$_n$ or B-[A-B]$_n$ or [A-B]$_n$ type in which A is a block comprising at least 50 % by weight of units deriving from styrene, B is a block comprising at least 50 % by weight of units deriving from ethyl (meth)acrylate, and n is a number greater than or equal to 1.

The blocks A and B are linked together linearly. If n = 1 the copolymer is an A-B-A or B-A-B triblock copolymer, or an A-B diblock copolymer. If n = 2, the copolymer is a A-B-A-B-A or B-A-B-A-B or A-B-A-B copolymer. If n ≥ 3, it is not a star or telechelic copolymer, in which a block A or a block B would make up the core. Preferably, is = 1, more preferentially the copolymer is an A-B-A triblock copolymer. It should be noted that the copolymer may comprise functional groups or polymerization groups or residues of such, functional or polymerization groups, at the end of macromolecular chains. They can be for example chain transfer groups, or residues of transfer groups, comprising for example a group of formula -S-CS-, or a residue of this group.

According to the particular embodiment mentioned above, block A comprises at least 50% by weight of units deriving from styrene. Block A may comprise units other than those deriving from styrene, which units may be intended for adjusting the properties of the copolymer or for facilitating its preparation. Block A may therefore be a random copolymer comprising units deriving from styrene and from other units. It is thus possible to adjust the solubility of block A in water or in other media, or to adjust its glass transition temperature and thus to adjust its rigidity. The other units of block A may be units deriving from monomers chosen from acrylic acid, methacrylic acid, methyl acrylate, butyl acrylate, ethylhexyl acrylate or 2-hydroxyethyl acrylate and from methyl methacrylate, butyl methacrylate, ethylhexyl methacrylate or 2-hydroxyethyl methacrylate. The presence of small quantities of methacrylic acid may especially facilitate preparation of the copolymer. Block A comprises preferably at least 75 wt%, preferably at least 90 wt%, and preferably at least 95 wt%, of units deriving from styrene.

In this case the possible other unit(s) deriving from monomers other than styrene represent therefore, preferably, 25 wt% or less, preferably 10 wt% or less, more preferably 5 wt% or less, even more preferentially about 2 wt%.

Block B as defined above comprises at least 50% by weight of units deriving from ethyl acrylate or from ethyl methacrylate. Block B may comprise units other than those deriving from ethyl acrylate or ethyl methacrylate, which units may be intended for adjusting the properties of the copolymer or for facilitating its preparation. Block B may therefore be a random copolymer comprising units deriving from ethyl acrylate or ethyl methacrylate and from other units. It is thus possible to adjust the solubility of block B in water or in other media, or to adjust its glass transtion temperature and thus to adjust its rigidity. The other units of block B may be units deriving from monomers chosen from acrylic acid, methacrylic acid, methyl acrylate, butyl acrylate, ethylhexyl acrylate, or 2-hydroxyethyl acrylate or from methyl methacrylate, butyl methacrylate, ethylhexyl methacrylate or 2-hydroxyethyl methacrylate. The presence of small quantities of methacrylic acid may especially facilitate preparation of the copolymer. Block B comprises preferably at least 75 wt%, preferably at least 90 wt%, and preferably at least 95 wt%, of units deriving from ethyl acrylate or ethyl methacrylate. In this case the possible other unit(s) deriving from monomers other than ethyl (meth)acrylate represent therefore, preferably, 25 wt% or less, preferably 10 wt% or less, and more preferably 5 wt% or less.

According to the invention, the number-average molecular weight of each block, whether it is a styrene unit block or an ethyl acrylate unit block, is between 1 000 g/mol and 200 000 g/mol, preferably between 5 000 g/mol and 100 000 g/mol.

It should be pointed out that the abovementioned average molecular weights are the theoretical or "target" average molecular weights of the block.

The theoretical average molecular weight $M_{block}$ of a block is typically calculated according to the equation below:

$$M_{block} = \sum_i M_i \frac{n_i}{n_{precursor}},$$

where $M_i$ is the molecular weight of a monomer i, $n_i$ is the number of moles of the monomers i, $n_{precursor}$ is the number of moles of the functional groups to which the macromolecular chain of the block will be linked. The functional groups may derive from a chain transfer agent (or chain transfer group) or an initiator, a preceding block. If it is from a preceding block, the number of moles may be considered to be the the number of moles of a compound to which the macromolecular chain of said preceding block has been linked, for example a chain transfer agent (or chain transfer group) or an initiator. In practice, the theoretical average molecular weights are calculated from the number of moles of monomers introduced and from the number of moles of precursor introduced.

The measured average molecular weight of a first block or of a copolymer denotes the number-average molecular weight in polystyrene equivalents of a block or of a copolymer measured by steric exclusion chromatography (SEC) in THF, calibrated using polystyrene standard. The measured average molecular weight of the nth block in a copolymer having n blocks is defined as the difference between the measured average molecular weight of the copolymer and the measured average molecular, weight of the copolymer having (n-1) blocks from which it was prepared.

A particularly advantageous block copolymer is a triblock copolymer comprising:

- a first block comprising units deriving from styrene having a number-average molecular weight of 30 000 g/mol;
- a second block composed of units deriving from ethyl acrylate, having a number-average molecular weight of 10 000 g/mol;
- a third block comprising units deriving from styrene having a number-average molecular weight of 30 000 g/mol.

A copolymer corresponding to the definition given above may be a copolymer for which the first block and/or the third block, and preferably the first block and the third block, comprise, besides the units deriving from styrene, units deriving from methacrylic acid, for example, in a (styrene/methacrylic acid) weight ratio of 98/2.

Another particularly advantageous copolymer is a triblock copolymer comprising:

- a first block comprising units deriving from styrene having a number-average molecular weight of 32 500 g/mol;
- a second block comprising units deriving from ethyl acrylate having a number-average molecular weight of 5.000 g/mol;
- a third block comprising units deriving from styrene having a number-average molecular weight of 32 500 μg/mol.

Another particularly advantageous copolymer is a triblock copolymer comprising:

- a first block comprising units deriving from styrene having a number-average molecular weight of 25 000 g/mol;
- a second block comprising units deriving from ethyl acrylate having a number-average molecular weight of 20 000 g/mol;
- a third block comprising units deriving from styrene having a number-average molecular weight of 25 000 g/mol.

These triblock copolymers may be in the form of an emulsion in water.

The block copolymers used according to the invention may be obtained by any known method, whether by radical polymerization that may or may not be controlled, by ring-opening (especially anionic or cationic) polymerization, by anionic or cationic polymerization, or even by chemical modification of a polymer.

Preferably, radical polymerization methods, known as living or controlled polymerization methods are used, and in a particularly preferred manner the controlled or living radical polymerization methods using a group transfer agent comprising a group of formula -S-CS-, especially those called RAFT or MADIX.

Mention may be made of, for example, anionic polymerization and controlled radical polymerization (see "New Methods of Polymer Synthesis", Blackie Academic & Professional, London, 1995, volume 2, page 1, or Trends Polym. Sci.4, page 183 (1996) by C.J. Hawker) which can be carried out according to various processes, for instance, atom transfer radical polymerisation (or ATRP) (see JACS, 117, page 5614 (1995), by Matyjasezwski, *et al.*)

By using the processes described above, it is possible to prepare a first block from monomers or from a mixture of monomers, initiators and/or polymerization control agents (group transfer agents containing -S-CS-), then the growth of a second block on the first block in order to obtain a diblock copolymer with different monomers from those used to prepare the preceding block, and possibly with addition of initiators and/or polymerization control agents, then the growth of a third block from the diblock copolymer in order to obtain a triblock' copolymer. These processes for preparing block copolymers are known to those skilled in the art. It may be mentioned that the copolymer may have as chain end a transfer group or residue of a transfer group, for example a group comprising an -S-CS- group (for example derived from a xanthate or a dithioester) or a residue of such a group.

Thus it is possible to prepare an A-B-A triblock copolymer according to the invention, by a process comprising the following steps:

- step a) preparation of block A by polymerization, preferably controlled radical polymerization, of a composition comprising:
- styrene;
- a source of free radicals; and
- at least one control agent, preferably an agent comprising an -S-CS- group for example a xanthate or a dithioester;
- step b) formation of the A-B diblock copolymer, by growth of block B onto block A, by polymerization, preferably controlled radical polymerization, of a composition comprising:
- ethyl acrylate or ethyl methacrylate; and
- possibly a source of free radicals;
- step c) formation of the A-B-A triblock copolymer, by growth of block A onto the A-B diblock, by polymerization, preferably controlled radical polymerization, of a composition comprising:
- ethyl acrylate or ethyl methacrylate; and
- possibly a source of free radicals;
- step d) possibly destruction or deactivation of a control agent group.

The polymerizations may be carried out in any suitable physical form, for example dissolved in a solvent, in emulsion in water (a "latex"process), in bulk, where appropriate by controlling the temperature and/or the pH so as to render some species liquid and/or soluble or insoluble.

The block copolymers used according to the invention are advantagously nonelastomeric copolymers.

The term "nonelastomeric copolymer" is understood to mean generally a copolymer that, when it is subjected to a stress intended to stretch it (for example by 30% relative to its initial length), does not return to a length approximately the same as its initial length when the stress is removed.

More specifically, the term "nonelastomeric copolymer" denotes a copolymer having an instantaneous recovery $R_i < 50\%$ and a delayed recovery $R_{2h} < 70\%$ after having undergone an elongation of 30%. Preferably, $R_i$ is < 30% and $R_{2h}$ is < 50%.

More precisely, the nonelastomeric character of the copolymer is determined according to the following protocol:

A copolymer film is prepared by casting a solution of the copolymer in a teflon-coated mold, then drying it for 7 days in a controlled environment at 23±5°C and 50±10% relative humidity.

A film of about 100 μm thickness is then obtained, from which rectangular test pieces are cut out (for example, using a punch) having a width of 15 mm and a length of 80 mm.

These samples in the form of test pieces are subjected to a tensile stress using a machine sold by Zwick, under the same temperature and humidity conditions as for the drying.

The test pieces are pulled at a rate of 50 mm/min and the distance between the jaws is 50 mm, which corresponds to the initial length ($l_0$) of the test piece.

The instantaneous recovery $R_i$ is determined in the following manner:

- the test piece is stretched by 30% ($\varepsilon_{max}$), that is to say about 0.3 times its initial length ($l_0$);
- the stress is released by unloading at a rate equal to the pull rate, namely 50 mm/min, and the residual elongation ($\varepsilon_i$) of the test piece is measured as a percentage, after the return to zero stress.

The instantaneous recovery $R_i$ (in %) is determined by the following equation:

$$R_i = ((\varepsilon_{max} - \varepsilon_i)/ \varepsilon_{max}) \times 100.$$

To determine the delayed recovery, the residual elongation of the test piece is measured as a percentage ($\varepsilon_{2h}$), two hours after the return to zero stress.

The delayed recovery $R_{2h}$ (en %) is given by the equation below:

$$R_{2h} = ((\varepsilon_{max} - \varepsilon_{2h})/ \varepsilon_{max}) \times 100.$$

The applicant has, in addition, demonstrated that although they are rigid enough to tighten the skin, the block copolymers used within the scope of the invention may advantagously have a weight ratio of units deriving from styrene

to units deriving from ethyl (meth)acrylate of greater than 1, preferably greater than 2. The ratio may also be greater than 5, or also even greater than 20.

The number-average molecular weight of the overall block copolymer is generally greater than 10 000 g/mol, preferably greater than 50 000 g/mol. This molecular weight does not preferably exceed 600 000 g/mol.

The weight-average molecular weight of the overall block copolymer is generally greater than 20 000 g/mol. It may also be greater than 100 000 g/mol and preferably below 1 000 000 g/mol.

Whatever its nature, synthetic polymer tightening agent may have a weight-average weight $M_w$ varying from 3 000 to 1 000 000 g/mol.

The synthetic polymer tightening agent is advantageously present in an effective-quantity in the composition used according to the invention. The term "effective quantify" is understood to mean in this case a quantity at least equal to the quantity needed to confer on the composition a tightening effect that is visible to the naked eye.

The synthetic polymer tightening agent may be present in the composition is a quantity of active material ranging from 0.01 to 20% by total weight of the composition, preferably from 1 % to 10% by weight relative to the total weight of the composition. The term "active material" is understood to mean the copolymer without solvent and devoid of the suspension medium resulting from the polymerization process.

The synthetic polymer tightening agent is combined in the compositions according to the invention with fibers.

## FIBERS

The aspect ratio, linear density and morphology of fibers are the three important factors for defining a fiber.

The fibers that can be used in the compositions acording to the invention may be short or long, individual or organized, for example braided. They are generally cylindrical unlike parallelepipedal platelets and spherical particles. They may have any morphology and especially a circular or polygonal (especially square, triangular, hexagonal or octogonal) cross section according to the specific application envisaged.

In particular, their ends may be blunt and/or polished to prevent injury. In particular, the fibers may have a length (L) ranging from 1 μm to 10 mm, preferably from 0.1 mm to 5 mm and better still from 0.1 mm to 1.5 mm. Their cross section may lie within a circle of diameter (D) ranging from 1 nm to 100 μm, preferably ranging from 1 μm to 50 μm and better still from 5 μm to 40 μm. Preferably, the fibers used according to the present invention have an aspect ratio, that is to say a L/D (length/diameter) ratio, ranging from 3.5 to 2500, better still from 5 to 500 and even better still from 5 to 150.

The fiber linear density is often given in denier or decitex. Denier is the weight in grams of 9 km of yarn. Preferably, the fibers used in the composition according to the invention have a linear density ranging from 0.15 to 30 denier, and better still from 0.18 to 18 denier.

The fibers that can be used in the composition acording to the invention may be hydrophilic or hydrophobic, mineral or organic fibers of synthetic or natural origin.

The fibers may be those used in the fabrication of textiles and especially fibers of silk, cotton, wool, linen, cellulose extracts, especially from wood, plants or algae, polyamide (Nylon®), modified cellulose (rayon, viscose, acetate, especially rayon acetate), poly(p-phenylene terephthalamide) especially Kevlar®, acrylic, especially polymethyl methacrylate or poly(2-hydroxyethyl methacrylate), polyolefin and especially polyethylene or polypropylene, glass, silica, aramid, carbon, especially in graphite form, polytetrafluoroethylene (especially Teflon®), insoluble collagen, polyester, polyvinyl chloride, polyvinylidene chloride, polyvinyl alcohol, polyacrylonitrile, chitosan, polyurethane, polyethylene phthalate, fibers formed from a blend of polymers such as those mentioned hereinabove, for instance polyamide/polyester fibers. As polyurethane fibers, mention may be made, for example, of poly(urethane-urea) polymer fibers, belonging to the class of elastanes, and especially those sold under the trademark LYCRA by DuPont.

The fibers that can be used in the composition acording to the invention are preferably chosen from polyamide fibers, poly(p-phenylene terephthalamide) fibers, cotton fibers and mixtures thereof.

The resorbable synthetic fibers used in surgery may also be employed, such as the fibers prepared from glycolic acid and caprolactone (MONOCRYL from Johnson & Johnson); the lactic acid/glycolic acid copolymer type resorbable synthetic fibers (VICRYL from Johnson & Johnson); terephthalic polyester fibers (ETHIBOND from Johnson & Johnson) and stainless steel threads (ACIER from Johnson & Johnson). Mixtures of the fibers mentioned above may also be used.

In addition, the fibers may or may not be surface-treated, and may be coated or uncoated. They may especially be coated and/or functionalized fibers. As coated fibers that can be used in the invention, mention may be made of polyamide fibers coated with copper sulfide to give an antistatic effect (for example R-STAT from Rhodia) or another polymer allowing a particular organization of the fibers (specific surface treatment) or a surface treatment leading to color/hologram effects (LUREX fiber from Sildorex, for example).

The fibers may also be functionalized, that is to say modified, especially surface-treated, so as to have a specific function or modified properties. This functionalization of the fibers may be carried out both on the fibers and in the fibers, and by any method for allowing a compound to be attached to the fibers or for trapping it in the cavities formed by the

geometry of the fibers. As methods, mention may be made of, for example, the coating of the fibers with an active agent; the attachment of particles containing an active agent, such as nanocapsules or nanospheres, to the fibers; adsorption in the fibers; fixing by chemical reaction. It is also possible to use fibers having particular functionalities, for example fibers that are UV-resistant by modification with chemical or physical sun screens; fibers that are bactericidal or antiseptic by modification with preservatives or antibacterial agents; fibers that are colored by modification with colorant molecules; fibers that are keratolytic or desquamating by modification with keratolytic or desquamating agents; fibers that are hydrating by modification hydrating agents or water-retaining polymers; fibers that are scented by modification with a fragrance; fibers that are analgesic or soothing by modification with an anti-inflammatory or a soothing agent; fibers that are antiperspirant by modification with an antiperspirant.

In particular, it is also possible to use polyamide fibers sold by Etablissements P. Bonte under the name POLYAMIDE 0.9 dtex 0.3 mm, having an average diameter of 15 to 20 μm, a linear density of about 0.9 dtex (0.81 denier) and a length ranging from 0.3 mm to 1.5 mm. It is also possible to use poly(p-hhenylene terephthalamide) fibers having an average diameter of 12 μm and a length of approximately 1.5 mm, such as those sold under the trademark KEVLAR FLOC by DuPont Fibers. These polyamide fibers are preferably introduced in an oily medium or by a dry route into a power. It is also possible to use cotton fibers having an average diameter of 20 μm, a length of 0.3 mm, and an aspect ratio of 15, such as those sold by Filature de Lomme, by the Institut Textile de France, by Textiles des Dunes or by Velifil.

Depending on their properties, the fibers used according to the present invention may be introduced into an aqueous medium, into an oily medium or into a powder.

In the same way, the fibers are preferably present in an effective quantity in the composition used according to the invention. In this case, the term "effective quantity" is understood to mean a quantity at least equal to the quantity needed so that the tightening effect is substantially persistent, that is to say still visible at least one hour, preferably at least two hours and better still at least five hours, after application of the composition.

Thus the fibers may be present in the composition according to the invention in an amount ranging from 0.1 to 50% by weight, preferably from 0.5 to 30% by weight, better still from 1 to 20% by weight and even better from 2 to 15%, or even from 2 to 10% by weight relative to the total weight of the composition.

The compositions according to the invention comprise, in addition to fibers and at least one tightening synthetic polymer, a physiologically acceptable medium.

## PHYSIOLOGICALLY ACCEPTABLE MEDIUM

The composition of the invention may be in any galenical form normally used for topical application to the skin, especially in the form of an aqueous, aqueous-acoholic or oily solution, an aqueous or oily gel, a liquid, paste or solid anhydrous product, a dispersion of oil in an aqueous phase in the presence of spherules, these spherules possibly being polymer nanoparticles such as nanospheres and nanocapsules or, better still, ionic and/or nonionic type lipid vesicles, a direct (O/W), inverse (W/O) or multiple (O/W/O and W/O/W) emulsion. The composition used according to the invention is preferably in the form of an aqueous gel or an oil-in-water emulsion.

For example, when it is applied to the skin, this composition may be more or less fluid and have the appearance of a white or coloured cream, a milk, a lotion, a serum, a paste or a foam. It may possibly be applied to the skin in the form of an aerosol. It may also be in solid form, and for example in stick or compact product form. It may be used as a skin care and/or make-up product. It may for example be used as a foundation. The composition may be an antiwrinkle composition.

As previously mentioned, the compositions used according to the invention may comprise an aqueous phase.

This aqueous phase may contain mostly water. It may also comprise a mixture of water and a water-miscible organic solvent (having a miscibility in water greater than 50% by weight at 25°C).

This aqueous phase may typically be present in an amount greater than or equal to 10%, preferably 30%, even more preferably 50%, or even 70% by weight relative to the total weight of the compositions.

In a known manner, the composition of the invention may also contain adjuvants common in the cosmetic field, such as hydrophilic or lipophilic gelling agents, hydrophilic or lipophilic active agents, preservatives, antioxidants, solvents, fragrances, fillers, sun screens, pigments, odour absorbers and dyes. The quantities of these various adjuvants are those conventionally used in the field under consideration, and, for example, are from 0.01 to 20% by total weight of the composition. These adjuvants, depending on their nature, may be introduced into the fatty phase, into the aqueous phase, into the lipid vesicles and/or into the nanoparticles. These adjuvants and also their concentrations must be such that they do not alter the sought-after property of the tightening agent.

When the composition of the invention is in an emulsion, the proportion of the fatty phase may range from 5 to 80% by weight, and preferably from 5 to 50% by weight relative to the total weight of the composition. The fatty substances, the emulsifiers and the coemulsifiers used in the emulsion composition are chosen from those conventionally used in the field under consideration. The emulsifier and the coemulsifier are preferably present, in the composition, in

a proportion ranging from 0.3 to 30% by weight, and preferably from 0.5 to 20% by weight relative to the total weight of the composition.

As fatty substances that can be used in the invention, mention may be made of oils and especially mineral oils (liquid petroleum), oils of plant origin (avocado oil, soybean oil), oils of animal origin (lanolin), synthetic oils (perhydrosqualene), silicone oils (cyclomethicone) and fluorinated oils (perfluoropolyethers). It is also possible to use, as fatty substances, fatty alcohols (cetylic alcohol), fatty acids, waxes and gums and, in particular, silicone gums.

As emulsifiers and coemulsifiers that can be used in the invention, mention may be made of, for example, fatty acid esters and polyethylene glycol esters such as PEG-50 stearate and PEG-40 stearate, and fatty acid esters and polyol esters such as glyceryl stearate and sorbitan.tristearate.

As hydrophilic gelling agents, mention may be made of, in particular, carboxyvinyl polymers (carbomers), acrylic copolymers such as acrylate/alkylacrylates copolymers, polyacrylamides, polysaccharides, natural gums and clays, and, as lipophilic gelling agents, mention may be made of modified clays like bentones, metal salts of fatty acids, hydrophobic silica and polyethylenes.

When it is used to diminish the signs of skin aging, the composition employed according to the invention may comprise, as active agents, at least one compound chosen from: desquamating and/or hydrating agents; depigmenting agents; antiglycation agents; agents for stimulating the synthesis of dermal or epidermal macromolecules and/or for preventing their decomposition; agents for stimulating the proliferation of fibroblasts and/or of keratinocytes or for stimulating the differentiation of keratinocytes; muscle relaxants; agents for combatting pollution and/or free radicals; and mixtures thereof

Examples of such active agents are: retinol and its derivatives such as retinyl palmitate; ascorbic acid and its derivatives such as magnesium ascorbyl phosphate and ascorbyl glucoside; tocopherol and its derivatives such as tocopheryl acetate; nicotinic acid and its precursors such as nicotinamide; ubiquinone; glutathion and its precursors such as L-2-oxothiazolidine-4-carboxylic acid; plant extracts and especially rock samphire and olive leaf extracts, and also the plant proteins and their hydrolysates such as rice or soybean protein hydrolysates; algal extracts and in particular laminaria extracts; bacterial extracts; sapogenins such as diosgenin and Dioscorea extracts, in particular wild yam extracts, comprising; $\alpha$-hydroxy acids; $\beta$-hydroxy acids such as salicylic acid and 5-n-octanoylsalicylic acid; oligopeptides and pseudodipeptides and their acylderivatives, in particular {2-[acetyl-(3-(trifluoromethyl)phenyl)amino]-3-methylbutyrylamino} acetic acid and the lipopeptides sold by Sederma under the trademarks MATRIXYL 500 and MATRIXYL 3000; lycopene; manganese and magnesium salts, in particular gluconates; and mixtures thereof

Furthermore, it is also possible to combine with the tightening agents used according to the invention other compounds known to those skilled in the art as tightening agents, especially plant proteins, polysaccharides of plant origin, possibly in the form of microgels, starches and mixed silicates.

When it is intended for smoothing the skin of the body, in particular as a slimming and/or firming composition, the composition used according to the invention may, as a variant, comprise one or more draining, lipolytic, disinfiltrating, slimming, firming, antiglycating and/or vasoprotective compounds.

Examples of such compounds may be chosen from: phosphodiesterase inhibitors such as caffeine and theobromine; monomethylsilanetriol mannuronate; tea, coffee, guarana, maté, or cola (*Cola nitida*) extracts; Ginkgo biloba extracts; horse chestnut extracts; Dioscorea extracts containing diosgenin; algal extracts and in particular *Laminaria digitata* extracts; and mixtures thereof.

In case of incompatibility, the active agents indicated above may be incorporated in spherules, especially notamment ionic or nonionic vesicles and/or nanoparticles (nanocapsules and/or nanospheres), so as to isolate them from each other in the composition.

## EXAMPLES

Example 1 : Synthesis of a tightening block polymer

A triblock copolymer was prepared comprising:

- a first block comprising units deriving from styrene having a number-average molecular weight of 30 000 g/mol;
- a second block composed of units deriving from ethyl acrylate having a number-average molecular weight of 10 000 g/mol;
- a third block comprising units deriving from styrene having a number-average molecular weight of 30 000 g/mol.

The procedure was based on a process that could be broken down into three distinct phases, a first step to obtain a polystyrene block, a second step to synthesize a polyethyl acrylate block following on from the first block and a third step to synthesize a polystyrene block following on from the second block, in order to obtain the polystyrene-b-poly(ethyl acrylate)-b-polystyrene triblock.

The synthesis of this copolymer was carried out in a 2-liter SVL type glass reactor. The maximum working volume of this type of reactor was 1.5 liters. The temperature inside the reactor was regulated by a Huber cryostat. The temperature was measured by a pt 100 probe immersed in the reactor and serving for regulation. The stirring unit was a stainless steel paddle. The rotation speed of the spindle was around 200 rpm. The reactor was also fitted with a reflux device (coil condensor) sufficiently effective to allow reflux of the monomers without product loss.
The process used was a latex-type emulsion polymerization process in water.

Step 1: Preparation of the first block.

This first step consisted in preparing the first block, which was made up of a styrene/methacrylic acid random copolymer, with a styrene/methacrylic acid weight ratio = 98/2 of the target theoretical mass: $M_n$ = 30 000 g/mol.
568.0 g of water, 12.0 g of sodium dodecyl sulfate and 0.95 g of sodium carbonate $Na_2CO_3$ were introduced, at room temperature, as feedstock. The mixture obtained was stirred for 30 minutes (200 rpm) under nitrogen. The temperature was then increased to 75°C, and then a mixture 1 was added, this mixture comprising:

- 25.71 g of styrene (St);
- 0.510 g of methacrylic acid (MAA); and
- 1.790 g of xantliate (CH3)($CO_2CH_3$)CH-S(C=S)$OCH_2CH_3$.

The mixture was brought to 85°C, then a solution of 0.390 g of ammonium persulfate $(NH_4)_2S_2O_8$ dissolved in 10.0 g of water was introduced.
After 5 minutes, the addition of a mixture 2 was started, this mixture comprising:

- 231.4 g of styrene (St); and
- 4.60 g of methacrylic acid (MAA).

The addition was continued for 115 minutes. After complete addition of the various ingredients, the copolymer emulsion obtained was kept at 85°C for two hours.
A sample (about 5 g) was then removed and analysed by steric exclusion chromatography (SEC) in THF. Its measured number-average molecular weight $M_n$ was equal to 26 600 g/mol as polystyrene equivalents (calibration by linear polystyrene standards). Its polydispersity index $M_w/M_n$ was equal to 2.0.
An analysis of the sample by gas chromatography revealed that the monomer conversion was greater than 99%.

Step 2: Preparation of the second block.

This second step consisted of the synthesis of an ethyl acrylate polymer.
The emulsion copolymer obtained above in step 1 was used as starting material, after having removed about 5 g for analysis and without having stopped the heating.
0.390 g of ammonium persulfate $(NH_4)_2S_2O_8$ diluted in 50.0 g of water were introduced continuously over one hour.
The following were added simultaneously over one hour at 85°C:

- 85.7 g of ethyl acrylate (EA).

The system was maintained at this temperature for a further two hours.
A sample (about 5 g) was then removed and analysed by steric exclusion chromatography (SEC) in THF. Its measured number-average molecular weight $M_n$ was equal to 37 000 g/mol as polystyrene equivalents (calibration by linear polystyrene standards). Its polydispersity index $M_w/M_n$ was equal to 1.9.
An analysis of the sample by gas chromatography revealed that the monomer conversion was greater than 99%.

Step 3: Preparation of the third block.

This third step consisted in preparing the third.block, which which was made up of a styrene/methacrylic acid random copolymer, with a styrene/methacrylic acid weight ratio = 98/2 of the target theoretical mass: $M_n$ = 30 000 g/mol.
The emulsion copolymer obtained above in step 2 was used as starting material, after having removed about 5 g for analysis and without having stopped the heating.
0.390 g of ammonium persulfate $(NH_4)_2S_2O_8$ diluted in 50.0 g of water were introduced continuously over three hours. Next, a mixture 3 was added simultaneously over three hours at 85°C, this mixture comprising:

- 50.0 g of water;
- 0.95 g of sodium carbonate $Na_2CO_3$.

Simultaneously, a mixture 4 was added, this mixture comprising:

- 257.1 g of styrene (St); and
- 5.14 g of methacrylic acid (MAA).

After complete addition of the various ingredients, the copolymer emulsion obtained was kept at 85°C for one hour.

Then 1.20 g of tert-butylbenzyl peroxide were introduced in a single go and the addition of a mixture 5 was started, this mixture comprising:

- 0.600 g erythorbic acid;
- 20.0 g of water.

The addition was continued for 60 minutes. After complete addition of the various ingredients, the emulsion was cooled to about 25°C over one hour.

A sample (about 5 g) was then removed and analysed by steric exclusion chromatography (SEC) in THF. Its measured number-average molecular weight $M_n$ was equal to 56 800 g/mol as polystyrene equivalents (calibration by linear polystyrene standards). Its polydispersity index $M_w/M_n$ was equal to 1.9.

An analysis of the sample by gas chromatography revealed that the monomer conversion was greater than 99.8 %.

The product obtained was a dispersion in water of the copolymer (latex), with a solids content of about 44 %.

<u>Example 2: antiwrinkle compositions.</u>

The examples of compositions below are given by way of illustation and are of a nonlimiting nature. In these examples, the compounds bear their INCI name.

The following examples 2A and 2B were prepared according to the procedure below.

Phase B (with the exception of ammonium polyacryldimethyltauramide) was heated to about 75°C. The ammonium polyacryldimethyltauramide was incorporated into the remainder of phase B which was stirred until a homogeneous gel was obtained.

Phase A was also heated to about 75°C and an emulsion was formed by incorporating this phase A into phase B.

When this emulsion had a temperature of 40-45°C, phase C and, where appropriate, (example 2B), phase D were incorporated therein. The emulsion was stirred and the stirring was maintained until the emulsion had cooled completely.

<u>Example 2A: Oil-in-water emulsion (comparative example).</u>

| Phase | Name | Concentration (wt%) |
|---|---|---|
| A | Glyceryl stearate (and) PEG-100 stearate (ARLACEL 165FL from Uniqema) | 2.00 |
| | Dimyristyl tartrate (and) cetearyl alcohol (and) C12-15 pareth-7 (and) PPG-25 laureth-25 (Cosmacol PSE from Sasol) | 1.50 |
| | Cyclohexasiloxane | 10.00 |
| | Stearyl alcohol | 1.00 |
| B | Water | qsp 100 |
| | Preservatives | 0.75 |
| | Pentasodium ethylenediamine tetramethylene phosphonate | 0.75 |
| | Ammonium polyacryldimethyltauramide (HOSTACERINE AMPS from Clariant) | 0.40 |
| | Xanthan gum (RHODICARE S from Rhodia) | 0.20 |

(continued)

| Phase | Name | Concentration (wt%) |
|---|---|---|
| C | PS(30000)-PEA(10000)-PS(30000) triblock polymer from example 1 | 16.10 |

Example 2B: antiwrinkle cosmetic composition: oil-in-water emulsion.

| Phase | Name | Concentration (wt%) |
|---|---|---|
| A | Glyceryl stearate (and) PEG-100 stearate (ARLACEL 165FL from Uniqema) | 2.00 |
| | Dimyristyl tartrate (and) cetearyl alcohol (and) C12-15 pareth-7 (and) PPG-25 laureth-25 (Cosmacol PSE from Sasol) | 1.50 |
| | Cyclohexasiloxane | 10.00 |
| | Stearyl alcohol | 1.00 |
| B | Water | qsp 100 |
| | Preservatives | 0.75 |
| | Pentasodium ethylenediamine tetramethylene phosphonate | 0.05 |
| | Ammonium polyacryldimethyltauramide (HOSTACERINE AMPS from Clariant) | 0.40 |
| | Xanthan gum (RHODICARE S from Rhodia) | 0.20 |
| C | PS(30000)-PEA(10000)-PS(30000) triblock polymer from example 1 | 16.10 |
| D | Polyamide fibers (Nylon-6,6 - Polyamide pulp 12185 from Paul Bonte) | 2.50 |

Example 3: Demonstration of the improvement in persistance properties of the formulations according to the invention.

The test consisted in applying a compressive stress, to breaking point, to a material (in this case the antiwrinkle creams of examples 2A and 2B) deposited on the surface of a soft and deformable foam. The use of this foam support made it possible to impose a significant deformation on the material deposited on the surface, and therefore to quantify its breaking strength. The compressive stress was exerted using a cylindrical punch of 1 mm diameter; the displacement rate of the punch was 0.1 mm/s. The test was carried out using a TA-XT2i texture analyzer sold by Stable Micro System. A curve of force F (in N) as a function of the displacement d (in mm) was thus obtained, from which it was possible to determine the breaking point of the material. Appended figure 1 shows such an example of a force versus displacement curve.

The parameter $W_{fracture}$ (the fracture energy in J/m$^2$) was adopted in order to quantify the strength of the material, this parameter corresponding to the area under the curve F = f(d)/punch area.

The substrate was made from a neoprene foam of 13 mm thickness. The material (antiwrinkle composition) was coated on this substrate so as to obtain, after drying for 24 h, a film with a thickness of 15 to 30 $\mu$m. The coating operations were carried out using a film-drawing device depositing 650 $\mu$m when wet.

The results obtained were the following:

| Material | $W_{fracture}$ (J/m$^2$) |
|---|---|
| Example 2A (comparative) | 21 $\pm$2 |
| Example 2B | 95$\pm$16 |

It was observed that the polyamide fibers used in the composition of example 2B had a reinforcing role, this reinforcing role being illustrated by an increase in the energy at break. It resulted in a more persistent tightening effect of the composition of example 2B. In fact, if the tightening effect is due to the formation of a rigid coating, it is understood that the longevity of this effect is due to the mechanical strength of this coating.

In has in addition been verified by a panel of six women that the composition of example 2B has a satisfactory tightening effect.

## Claims

1. A cosmetic skin care method for smoothing human skin on the face and/or body and/or to diminish or remove the signs of skin aging, in particular to reduce or remove wrinkles and/or fine lines from the skin comprising the topical application to the skin of a composition comprising, in a physiologically acceptable medium, at least:

    - one synthetic polymer tightening agent, and
    - fibers.

2. The method as claimed in the preceding claim, **characterized in that** said synthetic polymer tightening agent produces, at a concentration of 7 wt% in water or any physiologically acceptable medium, a shrinkage of isolated *stratum corneum* of at least 0.9% at 30°C and under 40% relative humidity.

3. The method as claimed in any one of the preceding claims, **characterized in that** said synthetic polymer tightening agent produces, at a concentration of 7 wt% in water or any physiologically acceptable medium, a shrinkage of isolated *stratum corneum* of more than 1% at 30°C and under 40% relative humidity.

4. The method as claimed in any one of the preceding claims, **characterized in that** said synthetic polymer tightening agent in said composition is either in solution or in suspension in a polar or apolar liquid, or is in dry form able to be redispersed in a cosmetic solvent.

5. The method as claimed in any one of the preceding claims, **characterized in that** said synthetic polymer tightening agent comprises at least one polyurethane polymer or copolymer, in particular a polyester/polyurethane copolymer or a polyether/polyurethane copolymer; an acrylic polymer or copolymer; or a grafted silicone polymer, or one of the blends thereof.

6. The method as claimed in any one of the preceding claims, **characterized in that** at least one synthetic polymer tightening agent is of interpenetrating polymer network (IPN) type.

7. The method as claimed in the preceding claim, **characterized in that** the IPN comprises at least one polyacrylic polymer and, where appropriate; at least one polyurethane or one vinylidene fluoride/hexafluoropropylene copolymer.

8. The method as claimed in claim 6, **characterized in that** the IPN comprises a blend of a polyurethane with a vinylidene fluoride/hexafluoropropylene copolymer.

9. The method as claimed in any one of the preceding claims, **characterized in that** said synthetic polymer tightening agent comprises a polymer in the form of a polycondensate.

10. The method as claimed in claim 9, **characterized in that** the polycondensate is chosen from acrylic polymers and copolymers, anionic, cationic, nonionic or amphoteric polyurethanes, polyurethane/acrylics, polyurethane/polyvinylpyrrolidones, polyester/polyurethanes, polyether/polyurethanes, polyureas, and blends thereof.

11. The method as claimed in any one of the preceding claims, **characterized in that** said synthetic polymer tightening agent comprises a grafted silicone polymer.

12. The method as claimed in the preceding claim, **characterized in that** the grafted silicone polymer is a polymer comprising a main silicone or polysiloxane (Si-O-polymer) chain onto which is grafted, within said chain and also possibly at one at least of its ends, at least one nonsilicone organic group.

13. The method as claimed in the preceding claim, **characterized in that** said nonsilicone organic group comprises at least one ethylenically unsaturated anionic monomer chosen from linear or branched, unsaturated carboxylic acids, possibly partially or completely neutralized in salt form and in particular acrylic acid, methacrylic acid, maleic acid, maleic anhydride, itaconic acid; fumaric acid and crotonic acid.

14. The method as claimed in any one of claims 11 to 13, **characterized in that** the grafted silicone polymer comprises in its structure the unit of formula (I) below:

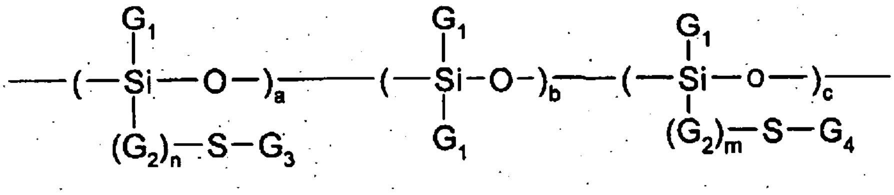

(I)

in which

- the $G_1$ radicals, which are identical or different, represent hydrogen or a $C_1$-$C_{10}$ alkyl radical or even a phenyl radical;
- the $G_2$ radicals, which are identical or different, represent a $C_1$-$C_{10}$ alkylene group;
- $G_3$ represents a polymer residue resulting from the (homo)polymerization of at least one ethylenically unsaturated anionic monomer;
- $G_4$ represents a polymers residue resulting from the (homo)polymerization of at least one ethylenically unsaturated hydrophobic monomer;
- m and n are, independently of one another, equal to 0 or 1; and
- a is an integer ranging from 0 to 50; b is an integer that may be between 10 and 350; and c is an integer ranging from 0 to 50, on condition that one of parameters a and c is other than 0.

**15.** The method as claimed in any one of claims 11 to 14, **characterized in that** the grafted silicone polymer is a polydimethylsiloxane (PDMS) onto which are grafted, via a thiopropylene-type linking chain, mixed polymer units of poly(meth)acrylic acid type and/or of polyalkyl (meth)acrylate type.

**16.** The method as claimed in any one of the preceding claims, **characterized in that** said synthetic polymer thightening agent comprises a polymer of "star" structure represented by formula (II) below:

$$A\text{-}[(M_1)_{p1} - (M_2)_{p2} \ldots (M_i)_{pj}]_n \qquad (II)$$

in which:

A represents, a multifunctional centre, of functionality "n", n being an integer greater than 2, in particular greater than 5;
- $[(M_1)_{p1} - (M_2)_{p2} \ldots (M_i)_{pj}]$ represents a polymer chain consisting of polymerized monomers $M_i$, which are identical or different, having a polymerization index pj, each branch being identical or different, and being covalently grafted onto said centre A; and
- i is greater than or equal to 1, and pj is,greater than or equal to 2,

said polymer comprising one or more monomers $M_i$, the corresponding homopolymer of which has a Tg of greater than or equal to 10 -°C, this or these monomer(s) $M_i$ being present in a minimum amount of 45% by weight relative to the total weight of all the monomers in the final polymer.

**17.** The method as claimed in any one of the preceding claims, **characterized in that** said synthetic polymer tightening agent comprises a block polymer.

**18.** The method as claimed in the preceding claim, **characterized in that** the block polymer bloc is a linear block copolymer of A-[B-A]., ou B-[A-B]$_n$ ou [A-B]$_n$ type in which n is a number greater than or equal to 1.

**19.** The method as claimed in the preceding claim, **characterized in that** A is a block comprising at least 50 % by weight of units deriving from styrene.

**20.** The method as claimed in claim 18 or 19, **characterized in that** B is a block comprising at least 50 % by weight of units deriving from ethyl (meth)acrylate.

**21.** The method as claimed in any one of claims 17 to 20, **characterized in that** the block copolymer is chosen from:

- triblock copolymers comprising:

  - a first block comprising units deriving from,styrene having a number-average molecular weight of 30 000 g/mol;
  - a second block composed of units deriving from ethyl acrylate having a number-average molecular weight of 10 000 g/mol;
  - a third block comprising units deriving from styrene having a number-average molecular weight of 30 000 g/mol;

- triblock copolymers comprising:

  - a first block comprising units deriving from styrene having a number-average molecular weight of 32 500 g/mol;
  - a second block comprising units deriving from ethyl acrylate having a number-average molecular weight of 5 000 g/mol;
  - a third block comprising units deriving from styrene having a number-average molecular weight of 32 500 g/mol; and

- triblock copolymers comprising:

  - a first block comprising units deriving from styrene having a number-average molecular weight of 25 000 g/mol;
  - a second block comprising units deriving from ethyl acrylate having a number-average molecular weight of 20 000 g/mol;
  - a third block comprising units deriving from styrene having a number-average, molecular weight of 25 000 g/mol.

**22.** The method as claimed in any one of the preceding claims, **characterized in that** said synthetic polymer tightening agent is present in the composition at a quantity of active material ranging from 0.01 to 20%, preferably from 1 to 10 %, by weight relative to the total weight of the composition.

**23.** The method as claimed in any one of the preceding claims, characterizes in that said synthetic polymer tightening agent has a weight-average weight Mw varying from 3 000 to 1 000 000 g/mol.

**24.** The method as claimed in any one of the preceding claims, **characterized in that** the fibers have a linear density ranging from 0.15 to 30 denier, and better still from 0.18 to 18 denier.

**25.** The method as claimed in any one of the preceding claims, **characterized in that** the fibers have an aspect ratio ranging from 3.5 to 2 500, better still from 5 to 500 , and even better still from 5 to 150.

**26.** The method as claimed in any one of the preceding claims, **characterized in that** the fibers have a length ranging from 1 μm to 10 mm, preferably from 0.1mm to 5 mm and better still from 0.1 mm to 1.5mm.

**27.** The method as claimed in any one of the preceding claims, **characterized in that** the fibers have a cross section that may lie within a circle of diameter ranging from 1 nm to 100 μm, preferably from 1 μm to 50 μm and better still from 5 μm to 40 μm.

**28.** The method as claimed in any one of the preceding claims, **characterized in that** the fibers are chosen from fibers of silk, cotton, wool, linen, cellulose, polyamide, modified cellulose (rayon, viscose, acetate, especially rayon acetate); poly(p-phenylene terephthalamide), acrylic, especially polymethyl methacrylate or poly(2-hydroxyethyl methacrylate), polyolefin and especially polyethylene or polypropylene, glass, silica, aramid, carbon, especially in graphite form, polytetrafluoroethylene, insoluble collagen, polyester, polyvinyl chloride, polyvinylidene chloride, polyvinyl alcohol, polyacrylonitrile, chitosan, polyurethane, polyethylene phthalate, lactic acid/glycolic acid copolymer, terephthalic polyester, fibers formed from a blend of polymers such as those mentioned hereinabove, for instance polyamide/polyester fibers, fibers prepared from glycolic acid and caprolactone and/or one of the mixtures thereof, stainless steel threads and mixtures thereof.

**29.** The method as claimed in the preceding claim, **characterized in that** the fibers are chosen from polyamide fibers,

poly(p-phenylene terephthalamide) fibers, cotton fibers and /or mixtures thereof.

**30.** The method as claimed in any one of the preceding claims, **characterized in that** the fibers are treated and/or coated fibers.

**31.** The method as claimed in any one of the preceding claims, **characterized in that** the fibers are present in an amount ranging from 0.1 to 50% by weight, preferably from 0.5 to 30% by weight, better still from 1 to 20% by weight and even better still from 2 to 15%, or even from 2 to 10% by weight relative to the total weight of the composition.

**32.** The method as claimed in any one of the preceding claims, **characterized in that** the composition is in serum, lotion, direct (O/W), inverse (W/O) or multiple (O/W/O and W/O/W) emulsion, stick or compact product form.

**33.** The method as claimed in any one of the preceding claims, **characterized in that** the composition is an antiwrinkle composition.

**34.** The method as claimed in any one of the preceding claims, **characterized in that** the composition is a foundation.

**Patentansprüche**

**1.** Kosmetisches Hautpflegeverfahren, um menschliche Haut im Gesicht und/oder auf dem Körper zu glätten und/oder um Anzeichen einer Hautalterung zu verringern oder zu beseitigen, insbesondere um Falten und/oder feine Linien auf der Haut zu verringern oder zu entfernen, das die topologische Anwendung einer Zusammensetzung auf die Haut umfasst, die in einem physiologisch annehmbaren Medium wenigstens enthält:

- ein synthetisches Polymer-Straffungsmittel und
- Fasern.

**2.** Verfahren nach dem vorhergehenden Anspruch, **dadurch gekennzeichnet, dass** das synthetische Polymer-Straffungsmittel bei einer Konzentration von 7 Gew.-% in Wasser oder irgendeinem physiologisch annehmbarem Medium eine Schrumpfung von isoliertem *Stratum Corneum* von wenigstens 0,9 % bei 30 °C und bei 40 % relativer Feuchtigkeit ergibt.

**3.** Verfahren nach einem der vorhergehenden Ansprüche, **dadurch gekennzeichnet, dass** das synthetische Polymer-Straffungsmittel bei einer Konzentration von 7 Gew.-% in Wasser oder irgendeinem physiologisch annehmbarem Medium eine Schrumpfung von isoliertem *Stratum Corneum* von mehr als 1% bei 30 °C und bei 40 % relativer Feuchtigkeit ergibt.

**4.** Verfahren nach einem der vorhergehenden Ansprüche, **dadurch gekennzeichnet, dass** das synthetische Polymer-Straffungsmittel in der Zusammensetzung entweder in Lösung oder in Suspension in einer polaren oder apolaren Flüssigkeit oder in trockener Form, die in einem kosmetischen Lösungsmittel wieder dispergiert werden kann, vorliegt.

**5.** Verfahren nach einem der vorhergehenden Ansprüche, **dadurch gekennzeichnet, dass** das synthetische Polymer-Straffungsmittel wenigstens ein Polyurethan-Polymer oder -Copolymer, insbesondere ein Polyester/Polyurethan-Copolymer oder ein Polyether/Polyurethan-Copolymer; und ein Acrylpolymer oder -Copolymer; oder ein Silikon-Pfropfpolymer oder eines der Gemische hiervon umfasst.

**6.** Verfahren nach einem der vorhergehenden Ansprüche, **dadurch gekennzeichnet, dass** wenigstens ein synthetisches Polymer-Straffungsmittel vom Typ durchdringendes Polymernetz (IPN) ist.

**7.** Verfahren nach dem vorhergehenden Anspruch, **dadurch gekennzeichnet, dass** das IPN wenigstens ein Polyacryl-Polymer umfasst und, falls geeignet, wenigstens ein Polyurethan oder ein Vinyliden-Fluorid/Hexafluorpropylen-Copolymer umfasst.

**8.** Verfahren nach Anspruch 6, **dadurch gekennzeichnet, dass** das IPN ein Gemisch aus Polyurethan mit einem Vinyliden-Fluorid/Hexafluorpropylen-Copolymer umfasst.

**9.** Verfahren nach einem der vorhergehenden Ansprüche, **dadurch gekennzeichnet, dass** das synthetische Polymer-Straffungsmittel ein Polymer in Form eines Polykondensats umfasst.

**10.** Verfahren nach Anspruch 9, **dadurch gekennzeichnet, dass** das Polykondensat gewählt ist aus Acryl-Polymeren und -Copolymeren, anionischen, kationischen, nichtionischen oder amphoterischen Polyurethanen, Polyurethan/Acrylen, Polyurethan/Polyvinylpyrrolidonen, Polyester/Polyurethanen, Polyether/Polyurethanen, Polyureas und Gemischen hiervon.

**11.** Verfahren nach einem der vorhergehenden Ansprüche, **dadurch gekennzeichnet, dass** das synthetische Polymer-Straffungsmittel ein Silikon-Pfropfpolymer umfasst.

**12.** Verfahren nach dem vorhergehenden Anspruch, **dadurch gekennzeichnet, dass** das Silikon-Pfropfpolymer ein Polymer ist, das eine Hauptsilikon- oder Polysiloxankette (Si-O-Polymer-Kette) umfasst, auf das innerhalb der Kette und außerdem möglicherweise an wenigstens einem seiner Enden wenigstens eine organische Nichtsilikongruppe gepfropft ist.

**13.** Verfahren nach dem vorhergehenden Anspruch, **dadurch gekennzeichnet, dass** die organische Nichtsilikongruppe wenigstens ein mit Ethylen ungesättigtes anionisches Monomer umfasst, das gewählt ist aus geraden oder verzweigten ungesättigten Carboxylsäuren, möglicherweise teilweise oder vollständig in Salzform neutralisiert, und insbesondere Acrylsäure, Methacrylsäure, Maleinsäure, Maleinsäureanhydrid, Itaconsäure, Fumarsäure und Crotonsäure.

**14.** Verfahren nach einem der Ansprüche 11 bis 13, **dadurch gekennzeichnet, dass** das Silikon-Pfropfpolymer in seiner Struktur die folgende Formeleinheit (I) enthält:

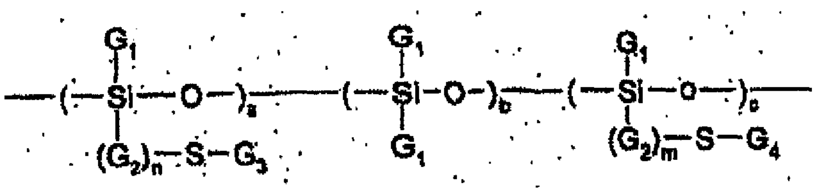

(I)

worin:

- die $G_1$-Radikale, die gleich oder verschieden sind, Wasserstoff oder ein $C_1$-$C_{10}$-Alklylradikal oder sogar ein Phenylradikal repräsentieren;
- die $G_2$-Radikale, die gleich oder verschieden sind, eine $C_1$-$C_{10}$-Alkengruppe repräsentieren;
- $G_3$ einen Polymerrest repräsentiert, der sich aus der (Homo) Polymerisierung wenigstens eines mit Ethylen ungesättigten anionischen Monomers ergibt;
- $G_4$ einen Polymerrest repräsentiert, der sich aus der (Homo) Polymerisierung wenigstens eines mit Ethylen ungesättigten hydrophoben Monomers ergibt;
- m und n unabhängig voneinander gleich 0 oder 1 sind; und
- a eine ganze Zahl im Bereich von 0 bis 50 ist; b eine ganze Zahl ist, die zwischen 10 und 350 liegen kann; und c eine ganze Zahl ist, die im Bereich von 0 bis 50 liegt, sofern einer der Parameter a und c von 0 verschieden ist.

**15.** Verfahren nach einem der Ansprüche 11 bis 14, **dadurch gekennzeichnet, dass** das Silikon-Pfropfpolymer ein Polydimethyl-Siloxan (PDMS) ist, auf das über eine Verbindungskette des Thiopropylen-Typs gemischte Polymereinheiten des Poly(meth)acrylsäure-Typs und/oder des Polyalkyl(meth)acrylat-Typs gepfropft sind.

**16.** Verfahren nach einem der vorhergehenden Ansprüche, **dadurch gekennzeichnet, dass** das synthetische Polymer-Straffungsmittel ein Polymer mit "Stern"-Struktur umfasst, das durch die folgende Formel (II) repräsentiert wird:

$$A\text{-}L(M_1)(M_2)_{p2}\ldots (M_i)_{pj}]_n \qquad (II)$$

worin:

- A ein multifunktionales Zentrum mit Funktionalität "n" repräsentiert, wobei n eine ganze Zahl größer als 2 und insbesondere größer als 5 ist;
- $[(M_1)_p-(M_2)_{p2} ... (M_i)_{pj}]$ eine Polymerkette repräsentiert, die aus polymerisierten Monomeren $M_i$ besteht, die gleich oder verschieden sind und einen Polymerisierungsindex pj haben, wobei jeder Zweig gleich oder verschieden ist, und die kovalent auf das Zentrum A gepfropft sind; und
i größer oder gleich 1 ist und pj größer oder gleich 2 ist,

wobei das Polymer ein oder mehrere Monomere $M_i$ enthält, dessen entsprechendes Homopolymer eine $T_g$ größer oder gleich 10 °C hat, wobei dieses Monomer oder diese Monomere $M_i$ in einer Mindestmenge von 45 Gew.-% in Bezug auf das Gesamtgewicht aller Monomere im endgültigen Polymer vorhanden ist (sind).

**17.** Verfahren nach einem der vorhergehenden Ansprüche, **dadurch gekennzeichnet, dass** das synthetische Polymer-Straffungsmittel ein Blockpolymer enthält.

**18.** Verfahren nach dem vorhergehenden Anspruch, **dadurch gekennzeichnet, dass** der Blockpolymer-Block ein lineares Blockpolymer des Typs $A-[B-A]_n$ oder $B-[A-B]_n$ oder $[A-B]_n$ ist, worin n eine Zahl größer oder gleich 1 ist.

**19.** Verfahren nach dem vorhergehenden Anspruch, **dadurch gekennzeichnet, dass** A ein Block ist, der wenigstens 50 Gew.-% Einheiten, die von Styrol abgeleitet sind, umfasst.

**20.** Verfahren nach Anspruch 18 oder 19, **dadurch gekennzeichnet, dass** B ein Block ist, der wenigstens 50 Gew.-% an Einheiten, die von Ethyl(meth)acrylat abgeleitet sind, umfasst.

**21.** Verfahren nach einem der Ansprüche 17 bis 20, **dadurch gekennzeichnet, dass** das Block-Copolymer ausgewählt ist aus:

- Triblock-Copolymeren, die umfassen:

  - einen ersten Block, der Einheiten enthält, die von Styrol abgeleitet sind und ein Zahlenmittel-Molekulargewicht von 30000 g/mol besitzen;
  - einen zweiten Block, der aus Einheiten gebildet ist, die von Ethylacrylat abgeleitet sind und ein Zahlenmittel-Molekulargewicht von 10000 g/mol besitzen;
  - einen dritten Block, der Einheiten enthält, die von Stryrol abgeleitet sind und ein Zahlenmittel-Molekulargewicht von 30000 g/mol besitzen;

- Triblock-Copolymeren, die umfassen:

  - einen ersten Block, der Einheiten enthält, die von Styrol abgeleitet sind und ein Zahlenmittel-Molekulargewicht von 32500 g/mol besitzen;
  - einen zweiten Block, der Einheiten enthält, die von Ethylacrylat abgeleitet sind und ein Zahlenmittel-Molekulargewicht von 5000 g/mol besitzen;
  - einen dritten Block, der Einheiten enthält, die von Styrol abgeleitet sind und ein Zahlenmittel-Molekulargewicht von 32500 g/mol. besitzen; und

- Triblock-Copolymeren, die umfassen:

  - einen ersten Block, der Einheiten enthält, die von Styrol abgeleitet sind und ein Zahlenmittel-Molekulargewicht von 25000 g/mol besitzen;
  - einen zweiten Block, der Einheiten enthält, die von Ethylacrylat abgeleitet sind und ein Zahlenmittel-Molekulargewicht von 20000 g/mol besitzen;
  - einen dritten Block, der Einheiten enthält, die von Styrol abgeleitet sind und ein Zahlenmittel-Molekulargewicht von 25000 g/mol besitzen.

**22.** Verfahren nach einem der vorhergehenden Ansprüche, **dadurch gekennzeichnet, dass** das synthetische Polymer-Straffungsmittel in der Zusammensetzung in einer Menge von aktivem Material im Bereich von 0,01 bis 20 Gew.-%, vorzugsweise im Bereich von 1 bis 10 Ges.-% in Bezug auf das Gesamtgewicht der Zusammensetzung vorhanden ist.

**23.** Verfahren nach einem der vorhergehenden Ansprüche, **dadurch gekennzeichnet, dass** das synthetische Polymer-Straffungsmittel ein Gewichtsmittel-Gewicht $M_w$ im Bereich von 3000 bis 1000000 g/mol besitzt.

**24.** Verfahren nach einem der vorhergehenden Ansprüche, **dadurch gekennzeichnet, dass** die Fasern eine lineare Dichte im Bereich von 0,15 bis 30 Denier und besser im Bereich von 0,18 bis 18 Denier haben.

**25.** Verfahren nach einem der vorhergehenden Ansprüche, **dadurch gekennzeichnet, dass** die Fasern ein Schlankheitsverhältnis im Bereich von 3,5 bis 2500, besser von 5 bis 500 und noch besser von 5 bis 150 haben.

**26.** Verfahren nach einem der vorhergehenden Ansprüche, **dadurch gekennzeichnet, dass** die Fasern eine Länge im Bereich von 1 mm bis 10 mm, vorzugsweise von 0,1 mm bis 5 mm und besser von 0,1 mm bis 1,5 mm haben.

**27.** Verfahren nach einem der vorhergehenden Ansprüche, **dadurch gekennzeichnet, dass** die Fasern einen Querschnitt haben, der innerhalb eines Kreises mit einem Durchmesser liegt, der von 1 nm bis 100 µm, vorzugsweise von 1 µm bis 50 µm und noch besser von 5 µm bis 40 µm, reicht.

**28.** Verfahren nach einem der vorhergehenden Ansprüche, **dadurch gekennzeichnet, dass** die Fasern gewählt sind aus Fasern aus Seide, Baumwolle, Wolle, Leinen, Zellulose, Polyamid, modifizierter Zellulose (Reyon, Viskose, Acetat, insbesondere Reyon-Acetat), Poly(p-phenylen-terephthalamid), Acryl, insbesondere Polymethyl-Metacrylat oder Poly(2-hydroxyethyl-methacrylat), Polyolefin und insbesondere Polyethylen oder Polypropylen, Glas, Siliciumdioxid, Aramid, Kohlenstoff, insbesondere in Graphitform, Polytetrafluorethylen, unlösliches Collagen, Polyester, Polyvinylchlorid, Polyvinylidenchlorid, Polyvinyl-Alkohol, Polyacrylonitril, Chitosan, Polyurethan, Polyethylen-Phthalat, Milchsäure/Glycolsäure-Copolymer, Terephthalat-Polyester, Fasern, die aus einem Gemisch von Polymeren wie etwa jenen, die oben erwähnt worden sind, gebildet sind, beispielsweise Polyamid/Polyester-Fasern, Fasern, die aus Glycolsäure und Caprolacton zubereitet sind, und/oder eines der Gemische hiervon, Edelstahlfasern und Gemische hiervon.

**29.** Verfahren nach dem vorhergehenden Anspruch, **dadurch gekennzeichnet, dass** die Fasern gewählt sind aus Polyamidfasern, Poly(p-phenylen-terephthalamid)-Fasern, Baumwollfasern und/oder Gemischen hiervon.

**30.** Verfahren nach einem der vorhergehenden Ansprüche, **dadurch gekennzeichnet, dass** die Faser behandelte und/oder beschichtete Fasern sind.

**31.** Verfahren nach einem der vorhergehenden Ansprüche, **dadurch gekennzeichnet, dass** die Fasern in einer Menge im Bereich von 0,1 bis 50 Gew.-%, vorzugsweise im Bereich von 0,5 bis 30 Gew.-%, besser von 1 bis 20 Gew.-% und noch besser von 2 bis 15 % oder sogar von 2 bis 10 Gew.-% in Bezug auf das Gesamtgewicht der Zusammensetzung vorhanden sind.

**32.** Verfahren nach einem der vorhergehenden Ansprüche, **dadurch gekennzeichnet, dass** die Zusammensetzung in Form eines Serums, einer Lotion, einer direkten (O/W), einer inversen (W/O) oder einer mehrfachen (O/W/O und W/O/W) Emulsion, eines Stift oder eines kompakten Produkts vorliegt.

**33.** Verfahren nach einem der vorhergehenden Ansprüche, **dadurch gekennzeichnet, dass** die Zusammensetzung eine Antifalten-Zusammensetzung ist.

**34.** Verfahren nach einem der vorhergehenden Ansprüche, **dadurch gekennzeichnet, dass** die Zusammensetzung ein Untergrund ist.

## Revendications

**1.** Procédé cosmétique de soin destiné à lisser la peau humaine du visage et/ou du corps et/ou à diminuer ou effacer les signes du vieillissement cutané, en particulier à réduire ou effacer les rides et/ou les ridules de la peau, comprenant l'application topique sur la peau d'une composition comprenant, dans un milieu physiologiquement acceptable, au moins :

- un agent tenseur polymérique synthétique, et
- des fibres.

2. Procédé selon la revendication précédente, **caractérisé en ce que** ledit agent tenseur polymérique synthétique produit, à une concentration de 7 % en poids dans l'eau ou tout milieu physiologiquement acceptable, une rétraction du stratum corneum isolé d'au moins 0,9 %, à 30 °C et sous une humidité relative de 40 %.

3. Procédé selon l'une quelconque des revendications précédentes, **caractérisé en ce que** ledit agent tenseur polymérique synthétique produit, à une concentration de 7 % en poids dans l'eau ou tout milieu physiologiquement acceptable, une rétraction du stratum corneum isolé de plus de 1 %, à 30 °C et sous une humidité relative de 40 %.

4. Procédé selon l'une quelconque des revendications précédentes, **caractérisé en ce que** ledit agent tenseur polymérique synthétique se trouve dans ladite composition, soit en solution ou en suspension dans un liquide polaire ou apolaire, soit sous forme sèche redispersable dans un solvant cosmétique.

5. Procédé selon l'une quelconque des revendications précédentes, **caractérisé en ce que** ledit agent tenseur polymérique synthétique comprend au moins un polymère ou copolymère de polyuréthane, en particulier un copolymère polyester-polyuréthanne ou un copolymère polyéther-polyuréthanne ; un polymère ou copolymère acrylique ; ou un polymère siliconé greffé, ou un de leurs mélanges.

6. Procédé selon l'une quelconque des revendications précédentes, **caractérisé en ce que** au moins un agent tenseur polymérique synthétique est de type réseaux de polymères interpénétrés (IPNs).

7. Procédé selon la revendication précédente, **caractérisé en ce que** l'IPN comprend au moins un polymère polyacrylique et le cas échéant au moins un polyuréthane ou un copolymère de fluorure de vinylidène et d'hexafluoroprapylène.

8. Procédé selon la revendication 6, **caractérisé en ce que** l'IPN comprend un mélange d'un polyuréthane avec un copolymère de fluorure de vinylidène et d'hexafluoropropylène.

9. Procédé selon l'une quelconque des revendications précédentes, **caractérisé en ce que** ledit agent tenseur polymérique synthétique comprend un polymère sous la forme d'un polycondensat.

10. Procédé selon la revendication 9, **caractérisé en ce que** le polycondensat est choisi parmi les polymères et copolymères acryliques, les polyuréthanes anioniques, cationiques, non ioniques ou amphotères, les polyuréthanes-acryliques, les polyuréthanes-polyvinylpyrrolidones, les polyester-polyuréthanes, les polyéther-polyuréthanes, les polyurées, et leurs mélanges.

11. Procédé selon l'une quelconque des revendications précédentes, **caractérisé en ce que** ledit agent tenseur polymérique synthétique comprend un polymère siliconé greffé.

12. Procédé selon la revendication précédente, **caractérisé en ce que** le polymère siliconé greffé est un polymère comprenant une chaîne principale de silicone ou polysiloxane (polymère de Si-O-) sur laquelle se trouve greffé, à l'intérieur de ladite chaîne ainsi qu'éventuellement à l'une au moins de ses extrémités, au moins un groupement organique non siliconé.

13. Procédé selon la revendication précédente, **caractérisé en ce que** ledit groupement organique non siliconé comprend au moins un monomère anionique à insaturation éthylénique choisi parmi les acides carboxyliques insaturés, linéaires ou ramifiés, éventuellement partiellement ou totalement neutralisés sous la forme d'un sel et en particulier l'acide acrylique, l'acide méthacrylique, l'acide maléique, l'anhydride maléique, l'acide itaconique, l'acide fumarique et l'acide crotonique.

14. Procédé selon l'une quelconque des revendications 11 à 13, **caractérisé en ce que** le polymère siliconé greffé comporte dans sa structure le motif de formule (I) suivant :

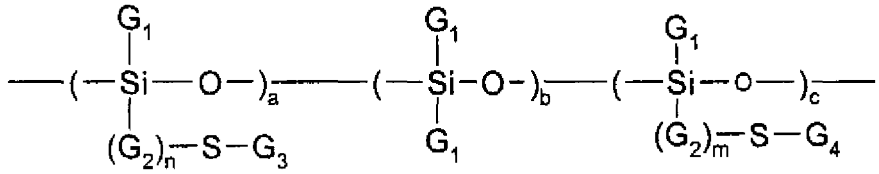

(I)

dans lequel

- les radicaux $G_1$, identiques ou différents, représentent l'hydrogène ou un radical alkyle en $C_1$-$C_{10}$ ou encore un radical phényle ;
- les radicaux G2, identiques ou différents, représentent un groupe alkylène en C1-C10 ;
- G3 représente un reste polymérique résultant de l'(homo)polymérisation d'au moins un monomère anionique à insaturation éthylénique ;
- G4 représente un reste polymérique résultant de l'(homo)polymérisation d'au moins un monomère hydrophobe à insaturation éthylénique ;
- m et n sont, indépendamment l'un de l'autre, égaux à 0 ou 1, et
- a est un nombre entier allant de 0 et 50 ; b est un nombre entier pouvant être compris entre 10 et 350, c est un nombre entier allant de 0 à 50 ; sous réserve que l'un des paramètres a et c soit différent de 0.

**15.** Procédé selon l'une quelconque des revendications 11 à 14, **caractérisé en ce que** le polymère siliconé greffé est un polydiméthylsiloxane (PDMS) sur lequel est greffé, par l'intermédiaire d'un chaînon de raccordement de type thiopropylène, des motifs polymères mixtes du type acide poly(méth)acrylique et/ou du type poly(méth)acrylate d'alkyle

**16.** Procédé selon l'une quelconque des revendications précédentes, **caractérisée en ce que** ledit agent tenseur polymérique synthétique comprend un polymère de structure en étoile représenté par la formule suivante (II) :

$$A\text{-}[(M_1)_{p1} - (M_2)_{p2} .... (M_i)p_i]_n \qquad (II)$$

dans laquelle :

- A représente un centre multifonctionnel, de fonctionnalité "n", n étant un entier supérieur à 2, en particulier supérieur à 5,
- $[(M_1)_{p1} - (M_2)_{p2} .... (M_i)_{pj}]$ représente une chaîne polymérique constituée de monomères Mi polymérisés, identiques ou différents, ayant un indice de polymérisation pj, chaque branche étant identique ou différente, et étant greffée de manière covalente sur ledit centre A,
- i est supérieur ou égal à 1, et pj est supérieur ou égal à 2, et

ledit polymère comprenant un ou plusieurs monomères $M_i$ dont l'homopolymère correspondant présente une Tg supérieure ou égale à 10 °C, ce ou ces monomères $M_i$ étant présents en une quantité minimale d'environ 45 % en poids par rapport au poids total de l'ensemble des monomères de polymère final.

**17.** Procédé selon l'une quelconque des revendications précédentes, **caractérisée en ce que** ledit agent tenseur polymérique synthétique comprend un polymère bloc.

**18.** Procédé selon la revendication précédente, **caractérisé en ce que** le polymère bloc est un copolymère blocs linéaire du type $A\text{-}[B\text{-}A]_n$ ou $B\text{-}[A\text{-}B]_n$ ou $[A\text{-}B]_n$ dans lesquels n est un nombre supérieur ou égal à 1.

**19.** Procédé selon la revendication précédente, **caractérisé en ce que** A est un bloc comprenant au moins 50 % en poids d'unités dérivant du styrène.

**20.** Procédé selon la revendication 18 ou 19, **caractérisé en ce que** B est un bloc comprenant au moins 50 % en poids d'unités dérivant du (méth)acrylate d'éthyle.

**21.** Procédé selon l'une quelconque des revendications 17 à 20, **caractérisé en ce que** le copolymère bloc est choisi parmi :

- les copolymères tribloc comprenant :

    - un premier bloc comprenant des unités dérivant du styrène ayant une masse moléculaire moyenne en nombre de 30 000 g/mol ;
    - un deuxième bloc constitué d'unités dérivant de l'acrylate d'éthyle ayant une masse moléculaire moyenne en nombre de 10 000 g/mol ;
    - un troisième bloc comprenant des unités dérivant du styrène ayant une masse moléculaire moyenne en nombre de 30 000 g/mol,

- les copolymères tribloc comprenant :

    - un premier bloc comprenant des unités dérivant du styrène ayant une masse moléculaire moyenne en nombre de 32 500 g/mol ;
    - un deuxième bloc comprenant des unités dérivant de l'acrylate d'éthyle ayant une masse moléculaire moyenne en nombre de 5 000 g/mol ;
    - un troisième bloc comprenant des unités dérivant du styrène ayant une masse moléculaire moyenne en nombre de 32 500 g/mol, et

- les copolymères tribloc comprenant :

    - un premier bloc comprenant des unités dérivant du styrène ayant une masse moléculaire moyenne en nombre de 25 000 g/mol ;
    - un deuxième bloc comprenant des unités dérivant de l'acrylate d'éthyle ayant une masse moléculaire moyenne en nombre de 20 000 g/mol ;
    - un troisième bloc comprenant des unités dérivant du styrène ayant une masse moléculaire moyenne en nombre de 25 000 g/mol.

**22.** Procédé selon l'une quelconque des revendications précédentes, **caractérisée en ce que** ledit agent tenseur polymérique synthétique est présent dans la composition en une quantité de matière active allant de 0,01 à 20 %, de préférence de 1 à 10 % en poids par rapport au poids total de la composition.

**23.** Procédé selon l'une quelconque des revendications précédentes, **caractérisé en ce que** ledit agent tenseur polymérique synthétique a une masse moyenne en poids Mw variant de 3 000 à 1 000 000 g/mol.

**24.** Procédé selon l'une quelconque des revendications précédentes, **caractérisé en ce que** les fibres ont un titre allant de 0,15 à 30 deniers, et mieux de 0,18 à 18 deniers.

**25.** Procédé selon l'une quelconque des revendications précédentes, **caractérisé en ce que** les fibres ont un facteur de forme allant de 3,5 à 2 500, mieux de 5 à 500 et encore mieux de 5 à 150.

**26.** Procédé selon l'une quelconque des revendications précédentes, **caractérisé en ce que** les fibres ont une longueur allant de 1 μm à 10 mm, de préférence de 0,1 mm à 5 mm et mieux de 0,1 mm à 1,5 mm.

**27.** Procédé selon l'une quelconque des revendications précédentes, **caractérisé en ce que** les fibres ont une section pouvant être comprise dans un cercle de diamètre allant de 1 nm à 100 μm, de préférence allant de 1 μm à 50 μm et mieux de 5 μm à 40 μm.

**28.** Procédé selon l'une quelconque des revendications précédentes, **caractérisé en ce que** les fibres sont choisies parmi les fibres de soie, de coton, de laine, de lin, de cellulose, de polyamide, de cellulose modifiée (rayonne, viscose, acétate notamment d'acétate de rayonne), de poly-p-phénylène téréphtalamide, en acrylique notamment de polyméthacrylate de méthyle ou de poly 2-hydroxyéthyl méthacrylate, de polyoléfine et notamment de polyéthylène ou de polypropylène, de verre, de silice, d'aramide, de carbone notamment sous forme graphite, de polytétrafluoroéthylène, de collagène insoluble, de polyesters, de polychlorure de vinyle ou de vinylidène, d'alcool polyvinylique, de polyacrylonitrile, de chitosane, de polyuréthane, de polyéthylène phtalate, de copolymère d'acide lactique et d'acide glycolique, de polyester téréphtalique, les fibres formées d'un mélange de polymères tels que ceux mentionnés ci-dessus, comme les fibres de polyamide/polyester, les fibres préparées à partir d'acide glycolique et de caprolactone et/ou un de leurs mélanges, les fils d'acier inoxydable et leurs mélanges.

**29.** Procédé selon la revendication précédente, **caractérisé en ce que** les fibres sont choisies parmi les fibres de polyamide, les fibres de poly-p-phénylène téréphtalamide, les fibres de coton et/ou leurs mélanges.

**30.** Procédé selon l'une quelconque des revendications précédentes, **caractérisé en ce que** les fibres sont des fibres traitées et/ou enrobées.

**31.** Procédé selon l'une quelconque des revendications précédentes, **caractérisé en ce que** les fibres sont présentes en une quantité allant de 0,1 à 50 % en poids, de préférence de 0,5 à 30 % en poids, mieux de 1 à 20 % en poids et encore mieux de 2 à 15 %, ou même de 2 à 10 % en poids par rapport au poids total de la composition.

**32.** Procédé selon l'une quelconque des revendications précédentes, **caractérisé en ce qu'**elle se présente sous la forme sous la forme d'un sérum, d'une lotion, d'une émulsion directe (H/E), inverse (E/H) ou multiple (H/E/H et E/H/E), d'un stick ou d'un produit compact.

**33.** Procédé selon l'une quelconque des revendications précédentes, **caractérisé en ce que** la composition est une composition anti-rides.

**34.** Procédé selon l'une quelconque des revendications précédentes, **caractérisé en ce que** la composition est un fond de teint.

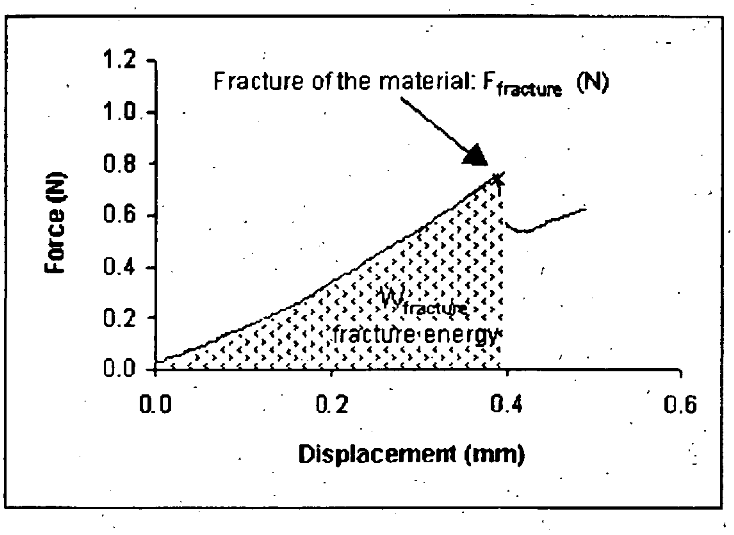
Force (N)
1.2
1.0
0.8
0.6
0.4
0.2
0.0
Fracture of the material: F fracture (N)
W fracture
fracture energy
0.0
0.2
0.4
0.6
Displacement (mm)

FIGURE 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1038519 A
- WO 9829092 A
- WO 2843025 A
- WO 03086342 A
- EP 1566171 A
- FR 2863494
- EP 1090626 A
- EP 1090627 A
- EP 1092424 A
- EP 1243251 A
- EP 1262168 A
- US 6139322 A
- US 6465001 B
- US 5349003 A
- EP 0582152 A
- WO 9323009 A
- WO 9503776 A
- EP 1043345 A

### Non-patent literature cited in the description

- New Methods of Polymer Synthesis. Blackie Academic & Professional, 1995, vol. 2, 1
- **C.J. Hawker.** *Trends Polym. Sci.,* 1996, vol. 4, 183
- **Matyjasezwski.** *JACS,* 1995, vol. 117, 5614